# EUROPÄISCHE PATENTSCHRIFT

(11) **EP 3 429 515 B1**
(45) Veröffentlichungstag und Bekanntmachung des Hinweises auf die Patenterteilung: **17.06.2020**
(21) Anmeldenummer: 17710278.7
(22) Anmeldetag: 13.03.2017
(51) Int. Cl.: A61F 5/01, A61F 5/058

(54) **ORTHOPÄDIETECHNISCHE EINRICHTUNG**
ORTHOPAEDIC DEVICE
DISPOSITIF ORTHOPÉDIQUE

(30) Priorität: 16.03.2016 DE 102016104877
(43) Veröffentlichungstag der Anmeldung: 23.01.2019
(73) Patentinhaber: Ottobock SE & Co. KGaA, 37115 Duderstadt (DE)
(72) Erfinder: KURZWEG, Annedore, 37085 Göttingen (DE); VAN DEN BERG, Emma Louise, 7511BM Enschede (NL); PLECHELMUS CHRISTENHUSZ, Harry, 48455 Bad Bentheim (DE)
(74) Vertreter: Gramm, Lins & Partner Patent- und Rechtsanwälte PartGmbB
(86) Internationale Anmeldenummer: PCT/EP2017/055861
(87) Internationale Veröffentlichungsnummer: WO 2017/157862

(56) Entgegenhaltungen:
- EP-A1- 0 121 718
- EP-A2- 1 452 154
- WO-A1-2009/134858
- DE-U1-202011 107 040
- US-A1- 2012 150 086
- US-A1- 2014 257 156

## Beschreibung

Die Erfindung betrifft eine orthopädietechnische Einrichtung mit einer Schaleneinrichtung, die in eine geschlossene Stellung, in der sie ein in der Schaleneinrichtung angeordnetes Körperteil zumindest teilweise umgreift, und in eine offene Stellung bringbar ist, in der das Körperteil in die Schaleneinrichtung bringbar ist, wobei die Schaleneinrichtung aus der offenen Stellung in die geschlossene Stellung bringbar ist, indem das Körperteil in die Schaleneinrichtung eingeführt wird, wobei die Einrichtung wenigstens ein Betätigungselement aufweist, das derart angeordnet und ausgebildet ist, dass es betätigt wird, wenn das Körperteil in die Schaleneinrichtung eingeführt wird, und das die Schaleneinrichtung aus der offenen Stellung in die geschlossene Stellung bringt, wobei das Betätigungselement ein Zugkraftübertragungselement aufweist.

Derartige orthopädietechnische Einrichtungen können beispielsweise Orthesen sein, die für unterschiedlichste Körperteile gedacht sein können. Dabei verfügen derartige Orthesen und andere orthopädietechnische Einrichtungen über eine Schaleneinrichtung, die zumindest teilweise um ein Körperteil, an dem die Orthese getragen werden soll, herumgelegt wird. So sind beispielsweise Fußheberorthesen oder Knöchelorthesen bekannt, die eine Schaleneinrichtung aufweisen, die um den Unterschenkel des Trägers der Orthese herumgelegt werden. Herkömmlicherweise werden dazu beispielsweise Gurte verwendet, die um das jeweilige Körperteil herum gelegt und anschließend geschlossen werden. Dies kann beispielsweise durch Klettverschlusselemente, Schnallen oder Druckknöpfe geschehen.

Die Schaleneinrichtung, beispielsweise ein Gurt, ist also in die geschlossene Stellung bringbar, in der das Körperteil oftmals vollständig, mindestens jedoch teilweise umgriffen wird, wodurch in der Regel erreicht wird, dass die orthopädietechnische Einrichtung am Körperteil des Trägers der Einrichtung befestigt wird. Um das Körperteil in die Schaleneinrichtung zu bringen oder es aus dieser herauszubewegen ist es jedoch notwendig, die Schaleneinrichtung in die offene Stellung zu bringen. Dies geschieht beispielsweise durch Lösen eines Klettverschlusses, durch Öffnen einer Schnalle oder auf andere aus dem Stand der Technik bekannte Weise.

Nachteilig ist jedoch, dass zumindest eine Hand, oftmals jedoch sogar beide Hände benötigt werden, um die Schaleneinrichtung in die geschlossene Stellung oder aus der geschlossenen Stellung in die offene Stellung zu bringen. Dies ist durch die oft schlecht zugängliche Positionierung der Schaleneinrichtung insbesondere für motorisch eingeschränkte Personen, beispielsweise Schlaganfallpatienten, schwierig. Dies gilt umso mehr, da oftmals auch die motorisch nicht bevorzugte Hand, bei Rechtshändern also beispielsweise die linke Hand, verwendet werden muss, um die Schaleneinrichtung aus der offenen Stellung in die geschlossene Stellung zu bringen. Insbesondere bei Unterarmorthesen oder Handgelenksorthesen ist dabei eine der Hände das Körperteil, an dem die Schaleneinrichtung anzuordnen ist, so dass diese Hand nicht zur Verfügung steht, um die Schaleneinrichtung in die geschlossene Stellung zu bringen.

Aus der DE 10 2013 019 079 A1 ist daher ein Verschlusssystem für ein orthopädisches Hilfsmittel, beispielsweise eine Fußheberorthese, bekannt, die mit einer Hand bedient werden kann. Ein Gurt, der um ein Körperteil herumgelegt werden soll, verfügt an seinem Ende über ein Element eines Magnetverschlusses. Das korrespondierende zweite Element ist an einem Teil der Orthese angeordnet, der mit dem Gurt verbunden werden soll. Da es sich um einen Magnetverschluss handelt, nimmt eine Anziehungskraft zwischen den beiden Elementen des Verschlusses zu, je näher sich die beiden Elemente kommen. Dadurch wird eine "Selbstfindungswirkung" des Verschlusses erreicht, die es erlaubt, den Verschluss mit nur einer Hand zu schließen.

Aus der DE 20 2011 107 040 U1 ist ein weiteres Verschlussmittel bekannt, dass es ermöglicht, eine orthopädische Stützeinrichtung mit nur einer Hand zu bedienen. Die dort beschriebene Einrichtung verfügt über eine Schaleneinrichtung, die aus zwei Schalen besteht, die durch ein Scharnier miteinander verbunden sind. Dabei erstreckt sich jede der beiden Schalen scherenartig auf beide Seiten des Scharniers, sodass im geöffneten Zustand zwei Enden der jeweiligen Schalensegmente nach innen in den Innenraum der Schaleneinrichtung hervorstehen. Wird nun beispielsweise ein Arm in die Schaleneinrichtung eingelegt, werden die beiden Scherenelemente betätigt und so die Schalen aufeinander zu bewegt.

Nachteilig ist jedoch, da eine Hand zum Schließen der Orthese benötigt wird und zudem die Möglichkeiten der Polsterung im Innern der Schaleneinrichtung begrenzt sind, da die scherenartig hervorstehenden Elemente relativ zueinander auch im Innenraum der Schaleneinrichtung bewegbar sein müssen. Daher kann es zu wenig komfortablen Situationen führen. Hinzu kommt, dass durch die scherenartige Aufspannung nur ein begrenzter Schwenkwinkel der beiden Schalenelemente relativ zueinander zur Verfügung steht und zudem das Körperteil beim Öffnen der Orthese durch die scherenartig hervorstehenden Vorsprünge aus der Orthese herausgedrückt wird, was ebenfalls zu einem wenig angenehmen Tragegefühl führen kann.

Aus der US 2010/0192288 A1 sind Polsterelemente bekannt, durch die Körperteile vor zu starker Druckeinwirkung geschützt werden sollen. Sie verfügen über eine Polsterlage, die sich an das zu schützende Körperteil anschmiegt, wenn es einen Druck auf die Polsterlage ausübt.

Die EP 1 452 154 A2, die als nächstliegender Stand der Technik angesehen wird, beschreibt eine Orthese, die als Verschluss eine vorgespannte Metallschiene aufweist, die daher aus einer ersten gestreckten Position in eine am Körper des Trägers anliegende Position wechseln kann.

Die US 2012/0150086 A1 zeigt eine Aufnahmeeinrichtung für ein Körperteil, die eine halbautomatisch wirkende Verschlusseinrichtung aufweist.

Der Erfindung liegt somit die Aufgabe zu Grunde, eine orthopädietechnische Einrichtung gemäß dem Oberbegriff des Anspruchs 1 so weiter zu entwickeln, dass sie leichter auch für motorisch eingeschränkte Personen gegebenenfalls ohne Hände anlegbar ist, wobei die vorbeschriebenen Nachteile vermieden oder zumindest reduziert werden.

Die Erfindung löst die gestellte Aufgabe durch eine orthopädietechnische Einrichtung gemäß dem Oberbegriff des Anspruchs 1, die sich dadurch auszeichnet, dass das Betätigungselement ein Zugkraftübertragungselement, insbesondere ein Band oder ein Tuch, aufweist, welches eine Länge aufweist, die kürzer ist als ein Innenumfang der Schaleneinrichtung in der geschlossenen Stellung, wobei die Länge vorzugsweise einstellbar ist. Es ist folglich nicht notwendig, mit einer oder zwei Händen die Schaleneinrichtung in die geschlossene Stellung zu bringen, indem beispielsweise Gurte um das Körperteil herumgelegt oder Schnallenvorrichtungen geschlossen werden müssen. Vielmehr ist es ausreichend, das Körperteil, an dem die orthopädietechnische Einrichtung angeordnet werden soll, in die Schaleneinrichtung, die sich in der offenen Stellung befindet, einzulegen. Allein dadurch wird die Schaleneinrichtung in die geschlossene Stellung gebracht, so dass das Anlegen auch für motorisch eingeschränkte Personen leicht möglich ist.

Die orthopädietechnische Einrichtung könnte beispielsweise eine Fußheberorthese sein, die in einem Schuh getragen wird. Sie verfügt über eine Fußplatte, die an der Unterseite des Fußes angeordnet ist und eine Schaleneinrichtung, die beispielsweise den Unterschenkel des Trägers der Orthese von vorn umgreift. Die Fußheberorthese kann im Schuh angeordnet werden und mit dem Schuh gemeinsam angelegt werden. Handelt es sich bei der Fußheberorthese um eine erfindungsgemäße orthopädietechnische Einrichtung muss der Träger der Orthese nach dem Anlegen des Schuhs lediglich den Unterschenkel in die dafür vorgesehene Schaleneinrichtung einführen, die allein dadurch in die geschlossene Position gebracht wird. Dadurch ist die Fußheberorthese gegebenenfalls bereits vollständig angelegt, sodass komplizierte Handhabungsschritte insbesondere an dieser für motorisch eingeschränkte Personen schwer zugänglichen Stelle des menschlichen Körpers entfallen.

Erfindungsgemäß weist die Einrichtung wenigstens ein Betätigungselement auf, das derart angeordnet und ausgebildet ist, dass es betätigt wird, wenn das Körperteil in die Schaleneinrichtung eingeführt wird, und dass die Schaleneinrichtung aus der offenen Stellung in die geschlossene Stellung bringt. Beim Einführen des Körperteils in die Schaleneinrichtung wird folglich das Betätigungselement betätigt. Dabei kann das Betätigungselement als Sensor oder Druckknopf ausgebildet sein. Erkennt beispielsweise ein Sensor, dass das Körperteil in der Schaleneinrichtung angeordnet ist, wird beispielsweise über eine elektrische Steuerung ein Steuersignal ausgesandt, durch das ein Aktuator betätigt wird, der beispielsweise die zueinander verschwenkbaren Schalenelemente aus der ersten Position in die zweite Position verschwenkt. Dies kann selbstverständlich auch durch einen Tastschalter oder Druckknopf geschehen, der durch das Körperteil betätigt wird, wenn es in die Schaleneinrichtung eingesetzt ist.

Nachteilig ist jedoch, dass diese Ausgestaltung mit erheblichem konstruktivem Aufwand verbunden ist und zudem eine Stromversorgung, beispielsweise in Form eines Akkus oder einer Batterie benötigt.

Erfindungsgemäß umfasst das Betätigungselement ein Zugkraftübertragungselement. Als besonders vorteilhaft hat sich die Verwendung eines Bandes oder eines Tuches herausgestellt. Dieses Zugkraftübertragungselement ist vorteilhafterweise an einem oder mehreren der Schalenelemente angeordnet. Insbesondere erstreckt es sich durch die Schaleneinrichtung derart, dass das Körperteil, das in die Schaleneinrichtung eingelegt oder eingeführt wird, mit dem Zugkraftübertragungselement in Kontakt kommt und es bewegt. Dadurch wird eine Zugkraft auf die beweglichen Schalenelemente oder das elastisch verformbare Schalenelement ausgeübt, durch die das jeweilige Schalenelement aus der ersten Position, in der sich die Schaleneinrichtung in der offenen Stellung befindet, in die zweite Position gebracht wird. Dadurch wird die Schaleneinrichtung geschlossen und das Körperteil zumindest teilweise umgriffen.

Vorzugsweise ist das Zugkraftübertragungselement in Umfangsrichtung der Schaleneinrichtung an mehreren Stellen mit der Schaleneinrichtung verbunden. Dies geschieht vorteilhafterweise über starre Abstandselemente, die jeweils eine Position der Schaleneinrichtung mit jeweils einer Position des Zugkraftübertragungselementes verbinden. Vorzugsweise ist das wenigstens eine Schalenelement der Schaleneinrichtung dabei elastisch ausgebildet, so dass es durch die Abstands-elemente, die starr ausgebildet sind, zu einer Verformung des wenigstens einen Schalenelementes kommt, wodurch eine optimale oder zumindest nahezu optimale Anpassung der Schaleneinrichtung und damit der orthopädietechnischen Einrichtung an das jeweilige Körperteil des Trägers der Einrichtung erreicht wird.

Vorzugsweise verfügt die Schaleneinrichtung über wenigstens zwei Schalenelemente, die relativ zueinander bewegbar sind und die relativ zueinander in einer ersten Position angeordnet sind, wenn sich die Schaleneinrichtung in der offenen Stellung befindet, und die in einer zweiten Position relativ zueinander angeordnet sind, wenn sich die Schaleneinrichtung in der geschlossenen Stellung befindet. Durch das Bewegen der wenigstens zwei Schalenelemente relativ zueinander wird folglich die Schaleneinrichtung aus der offenen Stellung in die geschlossene Stellung gebracht.

Vorteilhafterweise sind die Schalenelemente relativ zueinander verschwenkbar oder verschiebbar. Dazu können beispielsweise Scharniere, beispielsweise in Form eines Filmscharniers oder als separates Scharnierelement, vorhanden sein, die ein Verschwenken der Schalenelemente relativ zueinander ermöglichen.

In einer konstruktiv besonders einfachen Ausgestaltung der Schaleneinrichtung besteht diese lediglich aus zwei Schalenelementen, die miteinander verschwenkbar verbunden sind. Dabei kann ein bistabiles Scharniergelenk verwendet werden. Bistabile Gegenstände zeichnen sich durch zwei stabile Zustände aus, in die sie beispielsweise durch das Aufbringen mechanischer Energie oder einer Kraft überführt werden können. Ein bistabiles Scharnier kann beispielsweise in der geöffneten Position der beiden daran befestigten Schalenelemente vorgespannt sein, sodass es ohne äußere Krafteinwirkung nicht schließt. Wird jedoch durch ein Körperteil, das in die Schaleneinrichtung eingeführt, über das Zugkraftübertragungselement eine Kraft auf das Scharnier ausgeübt, wird es in den jeweils anderen stabilen Zustand überführt und schnappt beispielsweise in die geschlossene Position. Anstelle eines Scharniers kann auch ein bistabiles flächiges Element, beispielsweise band- oder blechförmiges Element verwendet werden, das ebenfalls bistabil vorgespannt ist und somit zwei stabile Positionen aufweist. Ähnliche Vorrichtungen sind beispielsweise als Reflektorbänder bekannt, die in einem gestreckten Zustand vorliegen können, indem sie eine parallel zur Längsachse verlaufende Wölbung aufweisen und in einen aufgewickelten Zustand überführt werden können, wenn auf das Element eine Kraft ausgeübt wird. Auch ein solches bistabiles flächiges Element, insbesondere aus einem Metall, gegebenenfalls mit einem Kunststoff ummantelt, kann für die Schaleneinrichtung verwendet werden.

Vorteilhafterweise ist eine wirksame Länge wenigstens eines Schalenelementes in Umfangsrichtung der Schaleneinrichtung einstellbar. Damit lässt sich der Umfang, den die Schaleneinrichtung in der geschlossenen Stellung aufweist, verändern und an die Bedürfnisse und Wünsche des Trägers der orthopädietechnischen Einrichtung anpassen. Dies kann auf verschiedene Weisen geschehen. So ist es beispielsweise denkbar, das wenigstens eine Schalenelement, dessen wirksame Länge veränderbar sein soll, durch mehrere, vorzugsweise zwei, gegeneinander verschiebliche Bauteile auszubilden, so dass durch eine Verschiebung der beiden Bauteile relativ zueinander die wirksame Länge angepasst werden kann. Dazu ist es von Vorteil, wenn eines der Bauteile über wenigstens ein Langloch verfügt, in dem ein Vorsprung des jeweils anderen Bauteils entlang gleiten kann. Dabei sind die beiden Bauteile relativ zueinander beispielsweise durch eine Schraub- oder Klemmverbindung vorzugsweise stufenlos zueinander fixierbar. Auf diese Weise lässt sich einfach, schnell und dennoch individuell die Länge des Schalenelementes an den Wunsch des Trägers anpassen. Alternativ oder zusätzlich dazu kann auch eine Verriegelungseinrichtung, die die orthopädietechnische Einrichtung in einer vorteilhaften Ausgestaltung aufweist, mit wenigstens einem ihrer Elemente an dem jeweiligen Schalenelement angeordnet sein. Durch eine Verschiebung der Position dieses wenigstens einen Elementes der Verriegelungseinrichtung lässt sich ebenfalls die wirksame Länge des Schalenelementes verändern.

Zusätzlich oder alternativ dazu verfügt die Schaleneinrichtung vorteilhafterweise über wenigstens ein elastisches Schalenelement, das elastisch verformt wird, wenn die Schaleneinrichtung aus der offenen Stellung in die geschlossene Stellung oder umgekehrt gebracht wird. Auch dieses elastische Schalenelement ist folglich in eine erste Position und in eine zweite Position bringbar, wobei sich die Schaleneinrichtung in der offenen Stellung befindet, wenn sich das elastische Element in der ersten Position befindet. Wird das elastische Element verformt und somit in die zweite Position gebracht, wird die Schaleneinrichtung geschlossen und befindet sich danach in der geschlossenen Stellung.

Vorteilhafterweise weist die Einrichtung wenigstens eine Verriegelungseinrichtung auf, die die Schaleneinrichtung selbsttätig in der geschlossenen Stellung halten. Dabei handelt es sich in einer bevorzugten Ausgestaltung um einen Magnetverschluss, der selbsttätig geschlossen wird, wenn die Schaleneinrichtung in die geschlossene Stellung gebracht wird. Um die Schaleneinrichtung in die geschlossene Stellung zu bringen, müssen wenigstens zwei Bereiche oder Bauteile der Schaleneinrichtung aufeinander zu bewegt werden. Dies können zwei Bereiche eines elastisch verformbaren Schalenelementes oder beispielsweise zwei relativ zueinander und gegebenenfalls relativ zum Rest der orthopädietechnischen Einrichtung bewegbar ausgebildete Schalenelemente sein. Ein Magnetverschluss kann dabei aus zwei Permanentmagneten oder einem Permanentmagneten und einem magnetisierbaren Element bestehen, die so angeordnet sind, dass in der geschlossenen Stellung der Schaleneinrichtung eine anziehende Kraft zwischen diesen beiden Elementen des Magnetverschlusses ausgeübt wird.

Wird beispielsweise durch das Zugkraftübertragungselement, beispielsweise ein Band, eine Zugkraft auf zwei verschwenkbare Schalenelemente ausgeübt, werden diese beim Einlegen des Körperteils in die Schaleneinrichtung aus der ersten Position in die zweite Position und damit die Schaleneinrichtung aus der offenen Stellung in die geschlossene Stellung gebracht. Dadurch werden die beiden Bauteile des Magnetverschlusses einander angenähert, so dass die attraktive Wechselwirkung, also die anziehende Kraft zwischen beiden Elementen, zunimmt. In einer bevorzugten Ausgestaltung sind die einzelnen Elemente des Magnetverschlusses so angeordnet, dass sie den Schließvorgang, der durch das Zugkraftübertragungselement begonnen wurde, unterstützen und gegebenenfalls vollenden, so dass die beiden Elemente des Magnetverschlusses durch die zwischen ihnen wirkende Anziehungskraft weiter aufeinander zu bewegt werden. Dies geschieht vorzugsweise bis sie in Kontakt kommen.

Die beiden Bauteile der Verriegelungseinrichtung, insbesondere des Magnetverschlusses, sind dabei vorzugsweise an unterschiedlichen Schalenelementen oder an unterschiedlichen Enden des gleichen Schalenelementes angeordnet, so dass durch das Schließen der Verriegelungseinrichtung auch die Schalenanordnung in den geschlossenen Zustand überführt wird. Durch eine Veränderung, beispielsweise Verschiebung, der Position wenigstens eines der Bauteile der Verriegelungseinrichtung an dem jeweiligen Schalenelement, an dem es angeordnet ist, lässt sich die wirksame Länge des Schalenelementes beeinflussen. Dadurch wird insbesondere eingestellt, wie weit die beiden Schalenelemente oder die Enden des gleichen Schalenelementes einander überlappen, wenn die Schaleneinrichtung im geschlossenen Zustand ist. Eine Verschiebung oder Versetzung der einzelnen Bauteile der Verriegelungseinrichtung kann auf unterschiedlichste Weise, beispielsweise über einen Verschiebemechanismus, über Klettverschlusselemente oder auf sonstige Weise geschehen.

Vorzugsweise handelt es sich bei der orthopädietechnischen Einrichtung um eine Orthese, insbesondere eine Armorthese oder eine Fuß- oder Beinorthese. Diese können vorteilhafterweise für einen Unterarm, für einen Knöchel oder einen Fuß verwendet werden. Selbstverständlich sind auch andere orthopädietechnische Einrichtungen möglich.

Das Zugkraftübertragungselement, das insbesondere als flächiges Element, beispielsweise Tuch, ausgebildet sein kann, verfügt vorzugsweise über Verstärkungs- und/oder Versteifungselemente, die für eine Versteifung des Zugkraftübertragungselementes entlang der Erstreckungsrichtung der Schaleneinrichtung sorgen. Dabei handelt es sich um die Richtung, in die sich auch das Körperteil erstreckt, das in die Schaleneinrichtung eingelegt wird.

Erfindungsgemäß hat das Zugkraftübertragungselement eine Länge, die kleiner ist als der Innenumfang der Schaleneinrichtung, wobei die Länge vorzugsweise einstellbar ist. Durch das Einlegen oder Einführen des Körperteils in die Schaleneinrichtung ist somit sichergestellt, dass eine Kraft auf das Zugkraftübertragungselement ausgeübt wird.

In einer bevorzugten Ausgestaltung verfügt die orthopädietechnische Einrichtung über wenigstens eine Verriegelungseinrichtung, die selbsttätig die Schaleneinrichtung in der geschlossenen Stellung hält, wobei die orthopädietechnische Einrichtung vorzugsweise nicht über weitere Verriegelungseinrichtungen verfügt, die die Schaleneinrichtung nicht selbsttätig in der geschlossenen Stellung halten. Solche Verriegelungseinrichtungen könnten beispielsweise in Form von Gurten, Schnallen oder Klettverschlusselemente vorhanden sein und müssen von Hand geschlossen werden. Daher werden sie im Rahmen der vorliegenden Erfindung nicht als Verriegelungseinrichtungen angesehen, die die Schaleneinrichtung selbsttätig in der geschossenen Stellung halten.

Auch wenn durch derartige Verriegelungselemente eine Verriegelung weiter verstärkt wird, hätten sie jedoch den Nachteil, dass die orthopädietechnische Einrichtung nicht mehr ohne Hände angelegt werden kann. Verfügt folglich die orthopädietechnische Einrichtung ausschließlich über eine oder mehrere Verriegelungseinrichtungen, die die Schaleneinrichtung selbsttätig in der geschlossenen Stellung halten, ist das Anlegen der orthopädietechnischen Einrichtung denkbar einfach, da lediglich das jeweilige Körperteil in die Schaleneinrichtung eingebracht werden muss. Allein dadurch wird die Schaleneinrichtung geschlossen und die orthopädietechnische Einrichtung an das Körperteil angelegt.

Eine orthopädietechnische Einrichtung im Sinne der vorliegenden Erfindung muss nicht zwangsläufig eine medizinische Indikation oder eine entsprechende Wirkung haben. Unter einer orthopädietechnischen Einrichtung im Sinne der vorliegenden Erfindung werden insbesondere auch unterstützende Einrichtungen verstanden, die lediglich den Komfort des Trägers der Einrichtung erhöhen sollen. So ist beispielsweise für Personen, die Arbeiten über Kopf ausführen müssen, eine orthopädietechnische Einrichtung hilfreich, durch die der auf Dauer ermüdende Einfluss der Schwerkraft auf die angehobenen Arme ausgeglichen oder zumindest abgemindert werden soll. Für derartige Arbeiten, die beispielsweise in der Fertigung aber auch bei Malerarbeiten oder sonstigen über Kopf durchzuführenden Arbeiten auftreten können, ist es von großem Nutzen, eine derartige orthopädietechnische Einrichtung zur Verfügung zu haben und diese zudem so einfach und schnell wie möglich anlegen zu können. Eine komplizierte Anordnung mittels mehrerer Verriegelungseinrichtungen, Gurte oder Bänder senkt die Akzeptanz der orthopädietechnischen Einrichtung und führt dazu, dass sie wenig oder schlimmstenfalls gar nicht mehr benutzt wird.

Mit Hilfe der beigefügten Zeichnungen wird nachfolgend ein Ausführungsbeispiel der vorliegenden Erfindung näher erläutert. Es zeigen:
- Figur 1 -: eine schematische Schnittdarstellung durch eine orthopädietechnische Einrichtung gemäß einem ersten Ausführungsbeispiel der Erfindung,
- Figur 2 -: durch eine Einrichtung gemäß einem zweiten Ausführungsbeispiel der vorliegenden Erfindung,
- Figuren 3a bis 3c -: unterschiedliche Stadien beim Anlegen einer orthopädietechnischen Einrichtung und
- Figuren 4a und 4b -: unterschiedliche Stadien beim Anlegen einer orthopädietechnischen Einrichtung gemäß einem weiteren Ausführungsbeispiel der vorliegenden Erfindung.

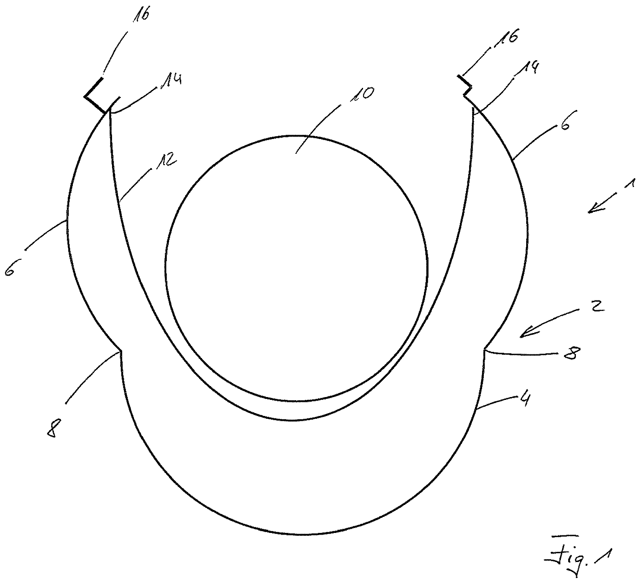

Figur 1 zeigt eine Schnittdarstellung durch eine orthopädietechnische Einrichtung 1 gemäß einem ersten Ausführungsbeispiel der vorliegenden Erfindung. Sie verfügt über eine Schaleneinrichtung 2, die über ein Hauptschalenelement 4 und zwei relativ dazu verschwenkbare Schalenelemente 6. Die Schalenelemente 6 sind über Scharniere 8 am Hauptschalenelement 4 schwenkbar gelagert.

Figur 1 zeigt die orthopädietechnische Einrichtung 1 mit der Schaleneinrichtung 2 in der offenen Stellung. Ein Arm 10, der das Körperteil darstellt, das in der Schaleneinrichtung 2 angeordnet werden kann, kann von oben durch die Öffnung zwischen den beiden Schalenelementen 6 eingeführt werden. Dabei wird er in Figur 1 nach unten bewegt, und kommt so mit einem Betätigungselement 12 in Kontakt dessen Endbereiche 14 an den Schalenelementen 6 befestigt ist. Wird nun der Arm 10 in Figur 1 weiter nach unten bewegt, wird auch auf das Betätigungselement 12 eine in diese Richtung gerichtete Kraft ausgeübt, die dafür sorgt, dass die beiden Schalenelemente 6 um die Scharniere 8 herum verschwenkt werden.

An den Enden der Schalenelemente 6 befinden sich zwei Verschlusselemente 16, die beispielsweise als Magnetelemente einer magnetischen Verriegelungseinrichtung ausgebildet sind. Durch die von dem Arm 10 über das Betätigungselement 12 auf die Schalenelemente 6 übertragene Kraft werden die beiden Verschlusselemente 16 aufeinander zu bewegt, bis sie miteinander in Kontakt kommen und so die Schaleneinrichtung 2 verschließen und ein versehentliches Öffnen verhindern.

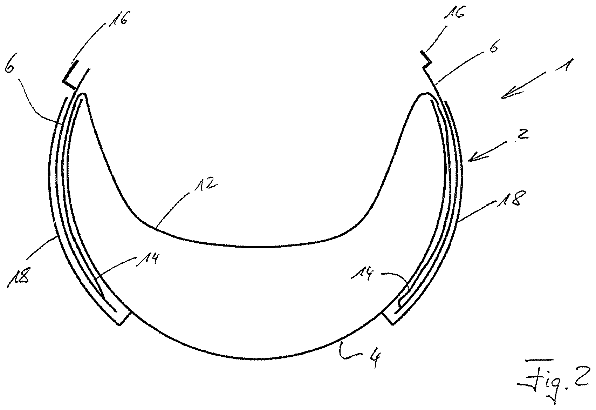

Figur 2 zeigt ein zweites Ausführungsbeispiel der orthopädietechnischen Einrichtung 1. Das Hauptschalenelement 4 verfügt über zwei Taschen 18, in denen jeweils ein Schalenelement 6 angeordnet ist. Diese sind nicht, wie im in Figur 1 gezeigten Ausführungsbeispiel über Scharniere 8 mit dem Hauptschalenelement 4 verbunden, sondern sind innerhalb der Taschen 18 verschiebbar angeordnet. Das Betätigungselement 12 erstreckt sich wie in Figur 1 durch die Schaleneinrichtung 2, so dass ein Körperteil, das in Figur 2 nicht gezeigt ist, eine Kraft auf das Betätigungselement 12 ausübt, wenn es in die Schaleneinrichtung 2 eingeführt wird. Da die Endbereiche 14 des Betätigungselementes 12 innerhalb der Taschen 18 an den Schalenelementen 6 angeordnet sind, führt eine derartige Kraft und eine Verschiebung des Betätigungselementes 12 in Figur 2 nach unten zu einem Verschieben der Schalenelemente 6 aus den Taschen 18 heraus. An den Enden der Schalenelemente 6 sind wieder die Verschlusselemente 16 zu erkennen, die Teile einer Verriegelungseinrichtung sind.

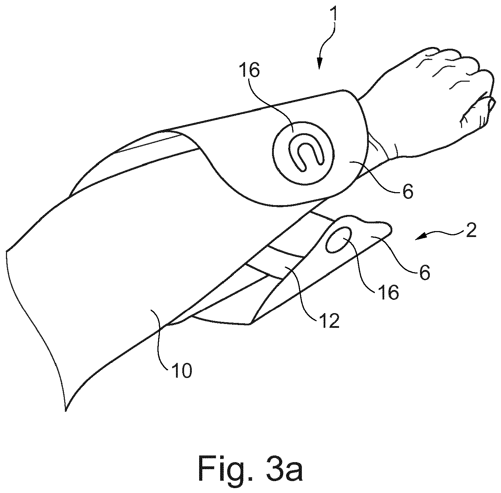

In den Figuren 3a bis 3c sind unterschiedliche Stadien beim Anlegen der orthopädietechnischen Einrichtung 1 gezeigt. Die Schaleneinrichtung 2 ist im gezeigten Ausführungsbeispiel vorgesehen, um den Arm 10 aufzunehmen. Sie verfügt über zwei Schalenelemente 6, zwischen denen ein Betätigungselement 12 angeordnet ist. In Figur 3a ist die Schaleneinrichtung 2 in der offenen Stellung gezeigt, der Arm 10 wurde gerade eingeführt. Man erkennt an den beiden Schalenelementen 6 Verschlusselemente 16, die im gezeigten Ausführungsbeispiel ein Magnetverschluss sind. Durch das Einlegen des Armes 10 in die Schaleneinrichtung 2 wird das Betätigungselement 12 betätigt und in diesem Fall eine Kraft auf die beiden Schalenelemente 6 ausgeübt, die dafür sorgt, dass die beiden Verschlusselemente 16 aufeinander zu bewegt werden.

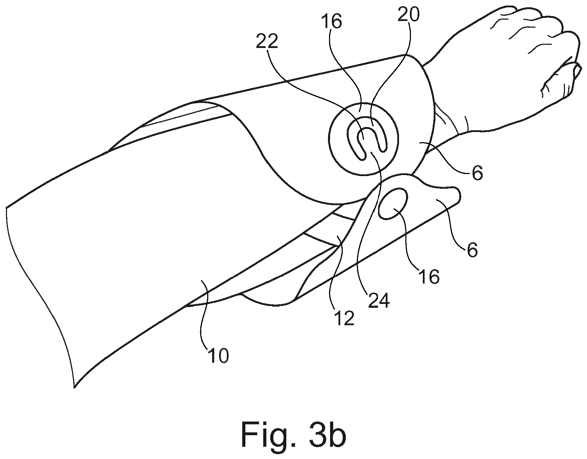

Dies ist in Figur 3b gezeigt. Die beiden Verschlusselemente 16 sind deutlich aufeinander zu bewegt worden, indem das Betätigungselement 12 betätigt wurde. Da die beiden Verschlusselemente 16 im gezeigten Ausführungsbeispiel magnetisch ausgebildet sind, herrscht zwischen Ihnen eine anziehende Kraft, die dafür sorgt, dass eine weitere Bewegung der beiden Verschlusselemente 16 aufeinander zu stattfindet. Im in Figur 3b oben gezeigten Verschlusselement 16 ist zu erkennen, dass dieses einen an einer Seite offenen Ring 20 aufweist, der als Erhöhung ausgebildet ist und folglich in seiner Mitte eine Vertiefung 22 aufweist. Diese verfügt in Figur 3b unten über eine Öffnung 24, durch die das in Figur 3b unten gezeigte Verschlusselement 16 eingeführt werden kann. Dies hat den Vorteil, dass bei geschickter Formgebung des Rings 20, der Vertiefung 22 und des unteren Verschlusselementes 16 es neben der magnetischen Haltekraft zu einer formschlüssigen Verriegelung kommt. Zum Öffnen der Verriegelungseinrichtung müssen die beiden Verschlusselemente 16 in genau der entgegengesetzten Richtung zueinander verschoben werden. Sofern dies nicht geschieht und eine Kraft in einer anderen Richtung aufgebracht wird, greift zusätzlich zur magnetischen Haltekraft die formschlüssige Verriegelung.

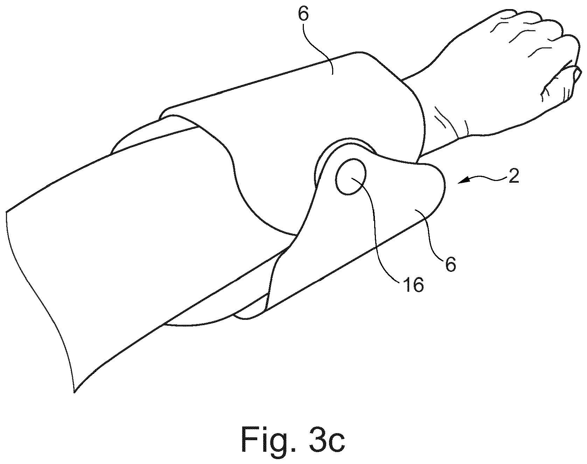

Dieses ist in Figur 3c dargestellt. Man erkennt die Schaleneinrichtung 2 in der geschlossenen Stellung die durch die beiden Verschlusselemente 16 weiter gesichert wird.

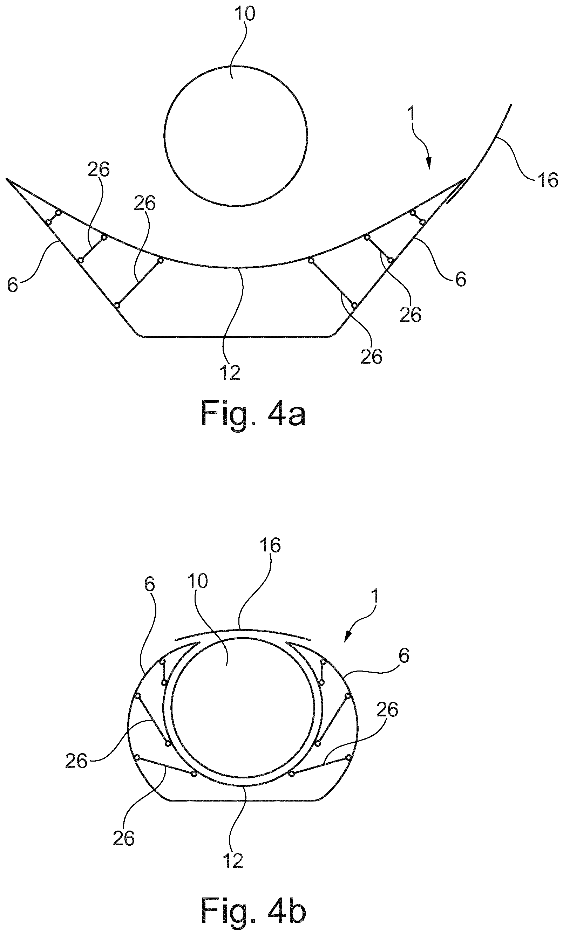

Figur 4a und 4b zeigt eine weitere Ausführungsform der orthopädietechnischen Einrichtung 1 im Querschnitt. Das Betätigungselement 12 ist wie bei den vorherigen Ausgestaltungen als Zugkraftübertragungselement ausgebildet. Die Vorrichtung verfügt über eine Schaleneinrichtung 2, die zwei Schalenelemente 6 aufweist, die in der in Figur 4a gezeigten offenen Stellung gerade und nicht gewölbt ausgebildet sind. Selbstverständlich können die Schalenelemente 6 jedoch auch eine Wölbung aufweisen. Zwischen dem Zugkraftübertragungselement 12 und den jeweiligen Schalenelementen 6 befinden sich Abstandselemente 26, die vorzugsweise starr ausgebildet sind und einen Abstand zwischen den beiden Bauteilen, die sie verbinden, konstant halten. Die Vorrichtung verfügt wieder über einen Verschlusselement 16.

Wird nun der Arm 10 oder ein anderes Körperteil des Träger des orthopädietechnischen Einrichtung 1, das in die Schaleneinrichtung 2 eingelegt werden soll, in die Schaleneinrichtung 2 eingelegt, sorgt dies bei der in Figur 4a und 4b gezeigten Ausführungsform dafür, dass die in Figur 4b gezeigte geschlossene Stellung erreicht wird. Durch die Abstandselemente 26 kann der sogenannte Finrayeffekt ausgenutzt werden, der für eine spezielle Wölbung der elastisch ausgebildeten Schalenelemente 6 sorgt. Das Verschlusselement 16 verschließt die verbleibende Öffnung zwischen den beiden Schalenelementen 6. Das Körperteil, in diesem Fall also der Arm 10, wird sicher umschlossen und die orthopädietechnische Einrichtung 1 kann ohne zusätzliche Zuhilfenahme einer Hand angelegt werden.

### Bezugszeichenliste

- 1: orthopädietechnische Einrichtung
- 2: Schaleneinrichtung
- 4: Hauptschalenelement
- 6: Schalenelement
- 8: Scharnier
- 10: Arm
- 12: Betätigungselement
- 14: Endbereich
- 16: Verschlusselement
- 18: Tasche
- 20: Ring
- 22: Vertiefung
- 24: Öffnung
- 26: Abstandelemente

## Patentansprüche

1. Orthopädietechnische Einrichtung (1) mit
einer Schaleneinrichtung (2), die
in eine geschlossene Stellung, in der sie ein in der Schaleneinrichtung (2) angeordnetes Körperteil (10) zumindest teilweise umgreift, und
in eine offene Stellung bringbar ist, in der das Körperteil (10) in die Schaleneinrichtung (2) bringbar ist,
wobei die Schaleneinrichtung (2) aus der offenen Stellung in die geschlossene Stellung bringbar ist, indem das Körperteil (10) in die Schaleneinrichtung (2) eingeführt wird, wobei die Einrichtung (1) wenigstens ein Betätigungselement (12) aufweist, das derart angeordnet und ausgebildet ist, dass es betätigt wird, wenn das Körperteil (10) in die Schaleneinrichtung (2) eingeführt wird, und das die Schaleneinrichtung (2) aus der offenen Stellung in die geschlossene Stellung bringt, wobei das Betätigungselement (12) ein Zugkraftübertragungselement aufweist, **dadurch gekennzeichnet, dass** das Zugkraftübertragungselement eine Länge aufweist, die kürzer ist als ein Innenumfang der Schaleneinrichtung in der geschlossenen Stellung.

2. Orthopädietechnische Einrichtung (1) nach Anspruch 1, **dadurch gekennzeichnet, dass** die Schaleneinrichtung (2) wenigstens zwei Schalenelemente (6) aufweist, die relativ zueinander bewegbar sind und die zueinander in einer ersten Position angeordnet sind, wenn sich die Schaleneinrichtung (2) in der offenen Stellung befindet, und in einer zweiten Position angeordnet sind, wenn sich die Schaleneinrichtung (2) in der geschlossenen Stellung befindet.

3. Orthopädietechnische Einrichtung (1) nach Anspruch 2, **dadurch gekennzeichnet, dass** die Schalenelemente (6) relativ zueinander verschwenkbar oder verschiebbar sind.

4. Orthopädietechnische Einrichtung (1) nach Anspruch 2 oder 3, **dadurch gekennzeichnet, dass** eine wirksame Länge wenigstens eines Schalenelementes in Umfangsrichtung der Schaleneinrichtung einstellbar ist.

5. Orthopädietechnische Einrichtung (1) nach einem der vorstehenden Ansprüche , **dadurch gekennzeichnet, dass** die Schaleneinrichtung (2) wenigstens ein elastisches Schalenelement aufweist, das elastisch verformt wird, wenn die Schaleneinrichtung (2) aus der offenen Stellung in die geschlossene Stellung oder umgekehrt gebracht wird.

6. Orthopädietechnische Einrichtung (1) nach einem der vorstehenden Ansprüche, **dadurch gekennzeichnet, dass** die Einrichtung (1) wenigstens eine Verriegelungseinrichtung aufweist, die die Schaleneinrichtung (2) selbsttätig in der geschlossenen Stellung hält.

7. Orthopädietechnische Einrichtung (1) nach Anspruch 6, **dadurch gekennzeichnet, dass** die Verriegelungseinrichtung wenigstens einen Magnetverschluss aufweist, der selbsttätig geschlossen wird, wenn die Schaleneinrichtung (2) in die geschlossene Stellung gebracht wird.

8. Orthopädietechnische Einrichtung (1) nach einem der vorstehenden Ansprüche, **dadurch gekennzeichnet, dass** die Einrichtung eine Orthese, insbesondere eine Armorthese, vorzugsweise für einen Unterarm, oder eine Fuß- oder Beinorthese, vorzugsweise eine Knöchel- oder Fußheberorthese ist.

9. Orthopädietechnische Einrichtung (1) nach einem der vorstehenden Ansprüche, **dadurch gekennzeichnet, dass** das Zugkraftübertragungselement wenigstens ein Versteifungselement aufweist, durch das eine Flexibilität des Zugkraftübertragungselementes entlang einer Erstreckungsrichtung der Schaleneinrichtung reduziert wird.

10. Orthopädietechnische Einrichtung (1) nach einem der vorstehenden Ansprüche, **dadurch gekennzeichnet, dass** das Zugkraftübertragungselement eine Länge aufweist, die einstellbar ist.

11. Orthopädietechnische Einrichtung (1) nach einem der vorstehenden Ansprüche, **dadurch gekennzeichnet, dass** das Zugkraftübertragungselement ein Band oder Tuch ist.

## Claims

1. Orthopaedic device (1) with
a shell mechanism (2) which
can be brought into a closed position, in which it engages at least partially around a body part (10) arranged in the shell mechanism (2), and
into an open position, in which the body part (10) can be brought into the shell mechanism (2),
wherein the shell mechanism (2) can be brought from the open position to the closed position by means of the body part (10) being introduced into the shell mechanism (2), wherein the device (1) has at least one actuation element (12) which is arranged and designed in such a way that it is actuated when the body part (10) is introduced into the shell mechanism (2), and it brings the shell mechanism (2) from the open position to the closed position, wherein the actuation element (12) has a tensile force transmission element, **characterized in that** the tensile force transmission element has a length that is shorter than the inner circumference of the shell mechanism in the closed position.

2. Orthopaedic device (1) according to claim 1, **characterized in that** the shell mechanism (2) has at least two shell elements (6), which can be moved relative to one another and are arranged relative to one another in a first position when the shell mechanism (2) is in the open position, and which are arranged in a second position when the shell mechanism (2) is in the closed position.

3. Orthopaedic device (1) according to claim 2, **characterized in that** the shell elements (6) can be swivelled or moved relative to one another.

4. Orthopaedic device (1) according to claim 2 or 3, **characterized in that** an effective length of at least one shell element in the circumferential direction of the shell mechanism can be adjusted.

5. Orthopaedic device (1) according to one of the above claims, **characterized in that** the shell mechanism (2) has at least one elastic shell element which experiences elastic deformation when the shell mechanism (2) is brought from the open position to the closed position or vice-versa.

6. Orthopaedic device (1) according to one of the above claims, **characterized in that** the device (1) has at least one locking device that automatically keeps the shell mechanism (2) in the closed position.

7. Orthopaedic device (1) according to claim 6, **characterized in that** the locking device comprises at least one magnetic lock which is automatically closed when the shell mechanism (2) is brought into the closed position.

8. Orthopaedic device (1) according to one of the above claims, **characterized in that** the device is an orthosis, in particular an arm orthosis, preferably for a lower arm, or a foot or leg orthosis, preferably an ankle or an ankle foot orthosis.

9. Orthopaedic device (1) according to one of the above claims, **characterized in that** the tensile force transmission element has at least one stiffening element, by means of which a flexibility of the tensile force transmission element along an extension direction of the shell mechanism is reduced.

10. Orthopaedic device (1) according to one of the above claims, **characterized in that** the tensile force transmission element has a length that is shorter than an inner circumference of the shell mechanism in the closed position, wherein the length is preferably adjustable.

11. Orthopaedic device (1) according to one of the above claims, **characterized in that** the tensile force transmission element is a band or a cloth.

## Revendications

1. Dispositif orthopédique (1) comportant
un dispositif formant coque (2) qui peut être amené dans
une position fermée dans laquelle il entoure au moins partiellement une partie du corps (10) disposée dans le dispositif formant coque (2),
une position ouverte dans laquelle la partie du corps (10) peut être amenée dans le dispositif formant coque (2),
dans lequel
le dispositif formant coque (2) peut être amené de la position ouverte vers la position fermée par introduction de la partie du corps (10) dans le dispositif formant coque (2),
le dispositif (1) comprend au moins un élément d'actionnement (12) qui est disposé et réalisé de manière à être actionné lorsque la partie du corps (10) est introduite dans le dispositif formant coque (2), et qui amène le dispositif formant coque (2) de la position ouverte vers la position fermée, l'élément d'actionnement (12) comprenant un élément de transmission d'une force de traction,
**caractérisé en ce que**
l'élément de transmission d'une force de traction présente une longueur inférieure à un pourtour intérieur du dispositif formant coque dans la position fermée.

2. Dispositif orthopédique (1) selon la revendication 1,
**caractérisé en ce que**
le dispositif formant coque (2) comprend au moins deux éléments de coque (6) qui sont mobiles l'un par rapport à l'autre et qui sont disposés dans une première position l'une par rapport à l'autre lorsque le dispositif formant coque (2) est en position ouverte, et qui sont disposés dans une seconde position lorsque le dispositif formant coque (2) est en position fermée.

3. Dispositif orthopédique (1) selon la revendication 2,
**caractérisé en ce que**
les éléments de coque (6) sont mobiles en basculement ou en translation l'un par rapport à l'autre.

4. Dispositif orthopédique (1) selon la revendication 2 ou 3,
**caractérisé en ce que**
une longueur efficace d'au moins un élément de coque est réglable en direction périphérique du dispositif formant coque.

5. Dispositif orthopédique (1) selon l'une des revendications précédentes, **caractérisé en ce que**
le dispositif formant coque (2) comprend au moins un élément de coque élastique qui est élastiquement déformé lorsque le dispositif formant coque (2) est amené de la position ouverte vers la position fermée ou inversement.

6. Dispositif orthopédique (1) selon l'une des revendications précédentes, **caractérisé en ce que**
le dispositif (1) comprend au moins un moyen de verrouillage qui maintient automatiquement le dispositif formant coque (2) dans la position fermée.

7. Dispositif orthopédique (1) selon la revendication 6,
**caractérisé en ce que**
le moyen de verrouillage comprend au moins un obturateur magnétique qui est automatiquement fermé lorsque le dispositif formant coque (2) est amené dans la position fermée.

8. Dispositif orthopédique (1) selon l'une des revendications précédentes, **caractérisé en ce que**
le dispositif est une orthèse, en particulier une orthèse pour bras, de préférence pour avant-bras, ou une orthèse pour pied ou pour jambe, de préférence une orthèse pour cheville ou pour muscle tibial antérieur.

9. Dispositif orthopédique (1) selon l'une des revendications précédentes, **caractérisé en ce que**
l'élément de transmission d'une force de traction comprend au moins un élément de rigidification par lequel une flexibilité de l'élément de transmission d'une force de traction est réduite le long d'une direction d'extension du dispositif formant coque.

10. Dispositif orthopédique (1) selon l'une des revendications précédentes, **caractérisé en ce que**
l'élément de transmission d'une force de traction présente une longueur qui est réglable.

11. Dispositif orthopédique (1) selon l'une des revendications précédentes, **caractérisé en ce que**
l'élément de transmission d'une force de traction est une bande ou une toile.
